# EUROPEAN PATENT SPECIFICATION

(11) **EP 0 869 812 B1**
(45) Date of publication and mention of the grant of the patent: **24.03.2004**
(21) Application number: 96942818.4
(22) Date of filing: 27.11.1996
(51) Int. Cl.: A61K 38/19, A61P 31/12

(54) **C-C CHEMOKINES THAT INHIBIT RETROVIRUS INFECTION**
C-C CHEMOKINE DIE DIE INFEKTION MIT RETOVIREN VERHINDERN
C-C CHEMOKINES INHIBANT L'INFECTION PAR RETROVIRUS

(30) Priority: 30.11.1995 US 7817 P
(43) Date of publication of application: 14.10.1998
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: LUSSO, Paolo, Garret Park, MD 20852 (US); GALLO, Robert C., Bethesda, MD 20817 (US); COCCHI, Fiorenza, Rockville, MD 20851 (US); DE VICO, Anthony, Alexandria, VA 22304 (US); GARZINO-DEMO, Alfredo, Washington, DC 20016 (US)
(74) Representative: VOSSIUS & PARTNER
(86) International application number: PCT/US1996/018993
(87) International publication number: WO 1997/019696

(56) References cited:
- WO-A-94/21277
- WO-A-94/24285
- WO-A-95/07715
- SCIENCE, vol. 270, 15 December 1995, pages 1811-1815, XP000616644 COCCHI F ET AL: "IDENTIFICATION OF RANTES, MIP-1ALPHA, AND MIP-1BETA AS THE MAJOR HIV-SUPPRESSIVE FACTORS PRODUCED BY CD8+ T CELLS"
- JOURNAL OF CLINICAL INVESTIGATION 97 (12). 1996. 2722-2727, XP000647539 BLUMAN E M ET AL: "Human natural killer cells produce abundant macrophage inflammatory protein-1-alpha in response to monocyte-derived cytokines."
- SCIENCE (WASHINGTON, D. C.) (1996), 274(5291), 1394-1395, 1996, XP002027715 COCCHI, FIORENZA ET AL: "Role of.beta.- chemokines in suppressing HIV replication. Reply to Comments"
- NAT. MED. (N. Y.) (1996), 2(11), 1244-1247, 1996, XP002027716 COCCHI, FIORENZA ET AL: "The V3 domain of the HIV -1 gp120 envelope glycoprotein is critical for chemokine -mediated blockade of infection"
- NATURE, vol. 381, no. 6584, 20 June 1996, pages 667-673, XP002027717 DRAGIC T. ET AL.: "HIV-1 entry into CD4+ cells is mediated by the chemokine recptor CC-CKR-5"
- NATURE, vol. 383, no. 6599, 3 October 1996, page 400 XP002027718 ARENZANA-SEISDEDOS F.ET AL.: "HIV blocked by chemokine antagonist"

## Description

### BACKGROUND OF THE INVENTION

Human immunodeficiency virus (HIV) induces a persistent and progressive infection leading, in the vast majority of the cases, to the development of the acquired immunodeficiency syndrome (AIDS). See Barrè-Sinoussi *et al., Science* **220**: 868 (1983); Popovic *et al., Science* **224**: 497 (1984); Gallo *et al., Science* **224:** 500 (1984); Sarngadharan *et al., Science* **224:** 504 (1994). During the phase of clinical latency, which lasts in some cases for more than 10 years, and in so-called "long-term non-progressors," the immune system appears to contain the levels of HIV replication effectively and thereby to minimize the pathogenic effects of the virus. Paul, *Cell* **82:** 177 (1995); Bolognesi, *Semin. Immunol.* **5**: 203 (1993).

As in other viral diseases, CD8 positive T lymphocytes are believed to play a critical role in the control of HIV infection. Whitton & Oldstone *in* VIROLOGY 369-81 (Raven Press 1990) ; Walker *et al., Nature* **328**: 345 (1987); Plata *et al., Nature:* 348 (1987); Nixon *et al.*, *Nature* **336**: 484 (1988); Letvin *et al.*, *Semin. Immunol.* **5**: 215 (1993); Walker *et al., Science* **234**: 1563 (1986); Brinchman *et al.*, *J. Immunol.* **144**: 2961 (1990) ; Castro *et al.,* Cell. *Immunol.* **132**: 246 (1991); Kanagi *et al., J. Immunol.* **119**: 470 (1988); Mackewicz & Levy, *AIDS Res. Hum. Retroviruses* **8**: 1039 (1992). Viral antigen-specific, major histocompatibility complex-restricted, CD8 positive cytotoxic T cells are present in the blood and tissues of both HIV-infected patients and simian immunodeficiency virus (SIV)-infected monkeys. Walker *et* *al .* (1987), *supra;* Plata *et al.* (1987), *supra;* Nixon *et al.* (1988), *supra*; Letvin *et al.*, Nature **5**: 215 (1993).

Another mechanism for the control of HIV infection may involve soluble factors with antiviral activity. Thus, certain cytokines, IL-10, and TNFα appear to have anti HIV activity. See Mackewicz & Levy (1992), *supra.* Moreover, activated CD8 positive T cells derived from HIV-infected individuals, as well as from HIV- or SIV-infected nonhuman primates; secrete soluble HIV-suppressive factor(s) (HIV-SF) which remain to be identified. In the literature, such factors are also sometimes referred to as "HIV-inhibitory factors" (HIV-IF). For example, see Walker *et al.* (1987), *supra.* The ability of CD8 positive T cells to produce HIV-IF is apparently not restricted to infected individuals, but has also been documented in healthy subjects. In the course of HIV infection, the *in vitro*-production of HIV-IF by isolated CD8 positive T cells seems to correlate with the disease stage, showing a progressive decline in parallel with the increasing deterioration of the immune system. See Mackewicz *et al.*, *J. Clin. Invest.* **87**: 1462 (1991).

Limited characterization of the SF activity was reported. As a partially purified fraction, it was denoted "CAF,'' for CD8 positive cell antiviral factor. The mechanism of action of CAF has been reported to be the inhibition of viral transcription. See WO 9507715 and EP 614372.

In spite of intensive efforts, the identity of the HIV-IF produced by CD8 positive T cells has not yet been elucidated nor their number determined, almost a decade after the first description of this activity. See Walker *et al.*, *Science* **234**: 1563 (1986); Brinchman *et al.*, *J.* Immunol. **144**: 2961 (1990). A major limitation in this respect has been the lack of reproducible and practical cellular systems for both the production and testing of HIV-IF. In fact, most of the cell lines commonly used for growing HIV *in vitro* are not suitable for HIV-IF testing, which is usually performed on primary viral isolates, grown directly in purified patient CD4 positive T cells, and employs autologous CD8 positive T cells as a source of HIV-IF. See Mackewicz *et al*. (1992), *supra.* Furthermore, the quantities of IF released by primary cells are low and inconsistent. As a consequence, no practical method for HIV-SF production is available for either experimental or clinical use.

Chemokines are a subgroup of immune factors and were shown to mediate chemotactic and other pro-inflammatory phenomena. See Schall, *Cytokine* **3**: 165 (1991). The chemokines are generally short peptides. The family of chemokines, intercrines, is subdivided into two distinct subfamilies, the C-X-C and C-C chemokines, according to the arrangement of the first two cysteines in their primary sequence.

The members of the C-C chemokines subfamily have remarkable similarities in their structural organization and biochemical properties. These homologies are consistent with the analogies observed in their biological effects, both *in vitro* and *in vivo.* For example, RANTES, MIP-1α and MIP-1β are all potent inducers of T-cell and mononuclear phagocyte chemotaxis, and exert diverse effects on eosinophilic and basophilic polymorphonuclear leukocytes. See Schall (1991), *supra.* For these properties, they have been suggested as possible mediators in autoimmune and allergic disorders.

Regarding their mode of action, several lines of evidence indicate that different C-C chemokines may use the same receptor moledule(s) on the cellular surface membrane, albeit with different binding affinities. *Id*. One such receptor, which binds RANTES and MIP-1α with high affinity, has been recently identified and cloned. See Neote *et al*., *Cell* **72**: 415 (1993); Gao *et al*., *J*. *Exp. Med.* **177**: 1421 (1993). RANTES, MIP-1α and MIP-1β genes have been isolated and expressed in heterologous systems. See Schall (1991), *supra*.

MIP-α was reportedly found to increase in concentration in the presence of HIV virion, and in turn appeared to inhibit infection. See Canque *et al.,* 9TH INTERNATIONAL CONGRESS OF IMMUNOLOGY, page 115 (1995) Abstract No. 1907; and Canque *et al.*, *Blood:* 84(10) Supp. 1 (1994). But other reports do not find MIP-1α to be present in increased levels during infection. See Sharma *et al.*, *Biochem. & Biophys. Res. Comm.* 208 (2): 704-13 (1995).

### SUMMARY OF THE INVENTION

Pursuant to the present invention, three chemokines, RANTES, macrophage inflammatory protein 1α (MIP-1α) and macrophage inflammatory protein 1β (MIP-1β), are identified as factors which can inhibit the spread of HIV. In relation to several criteria, these chemokines differ from CD8 positive lymphocyte-produced antiviral factor (CAF). Most importantly, CAF is only a partially characterized activity, with features which generally do not distinguish them from other cytokines. By contrast, RANTES, MIP-1α and MIP-1β are well-characterized chemokines, are available commercially, as are antibodies and assays for their detection, and their respective genes have been isolated. Furthermore, unlike CAF, the chemokines in question do not act at the level of HIV transcription. Also in contrast with CAF, RANTES induces CD4 positive lymphocyte activation. See Bacon *et al.*, *Science* **269**: 1727 (1995). Finally, these C-C chemokines are inactive against some HIV-1 isolates (*e.g.*, HIV_{MB}) whereas the CAF activity was reportedly active against all HIV isolates. See Kannagi *et al.*, *J. Virol.* **64**: 3399 (1990). In a further contrast between CAF and the presently identified, antiviral chemokines, CAF reportedly is produced by CD8 positive T lymphocytes, while RANTES, MIP-1α and MIP-1β are produced in a variety of cellular systems. See Schall (1991), *supra*.

The ability of RANTES, MIP-1α and MIP-1β to inhibit the spread of HIV informs treatment procedures that utilize one of more of the purified chemokines, either in native or modified form. The chemokines can be overexpressed and purified from an expression system other than induced, CD8 positive cells. Thus, their production and purification would be simpler and cheaper and the factor could be purified without risking contamination by human pathogens. Genetic therapy and prophylactic use of the gene is also enabled. The presence of the chemokines can serve as a diagnostic marker for HIV infection.

In accordance with one aspect of the current invention, therefore, a therapeutic preparation has been provided that comprises any combination of two or more chemokines selected from a group consisting of RANTES, MIP-1α, and MIP-1β. In a preferred embodiment, the amino-acid sequence of any of those chemokines is altered such that the protein domain that causes inflammation is affected.

In accordance with a second aspect of the current invention, a therapeutic preparation has been provided that comprises a chemokine analog selected from the group of a RANTES analog, a MIP-1α analog, and a MIP-1β analog. In a preferred embodiment, the retrovirus is HIV. In another preferred embodiment, the administration is effected via *in vivo* expression of a polynucleotide encoding the chemokine. In yet another preferred embodiment, the polyhucleotide has a sequence that reflects a modification in relation to a natural gene coding for the chemokine. In still another preferred embodiment, the modification is (i) a tissue-specific promoter, (ii) an inducible promoter, or (iii) an altered coding sequence such that a protein domain that causes inflammation is affected. In a further preferred embodiment, the treatment of retroviral infections is prophylactic, comprising a step of administering to an organism a chemokine selected from a group consisting of RANTES, MIP-1α, and MIP-1β.

In accordance with a third aspect of the current invention, there has been provided a method for isolating retrovirus from an infected subject, comprising the steps of (A) administering to a sample an antibody that binds RANTES, MIP-1α or MIP-1β and then (B) cocultivating the sample with uninfected lymphocytes.

### BRIEF DESCRIPTION OF THE DRAWINGS

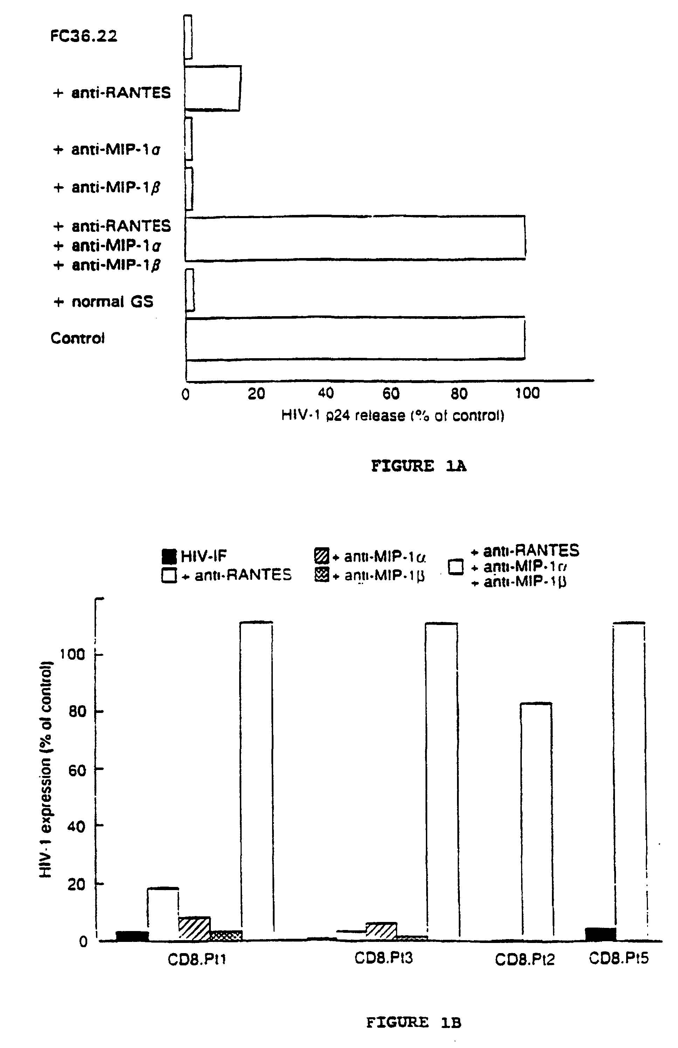

Figures 1A and 1B show neutralization of the HIV-SF activity contained in the culture supernatant of clone FC36.22 (**A**) or of activated CD8 positive T cells from HIV-infected patients (**B**) by polyclonal goat IgG neutralizing antibodies (NAb) against RANTES, MIP-1α and MIP-1β. The PM1/HIV-1_{BaL} acute test system was used to measure HIV-SF activity -- see Detailed Description of Preferred Embodiments section. Neutralization was performed by incubating the HIV-SF containing culture supernatant with NAb, alone or in combination, for 30 min at room temperature. As control, untreated culture supernatant was handled in parallel. Also as a control, cells were treated with NAb without added supernatant. Anti-RANTES, anti-MIP-1α and anti-MIP-1β NAb were used at 200, 50 and 100 µg/ml, respectively. The IgG fraction was purified from a non-immune goat serum (normal GS) and used as a control, at 200 µg/ml. Cell-free supernatant were collected at day 3, filtered and stored frozen at -70°C until use. The proportion of patient CD8 positive T-cell culture supernatant used (20% [vol/vol] for patient 1, 40% for patient 3, 75% for patients 2 and 5) was previously determined as the approximate ED₉₅ for HIV-1_{BaL} infection in PM1.

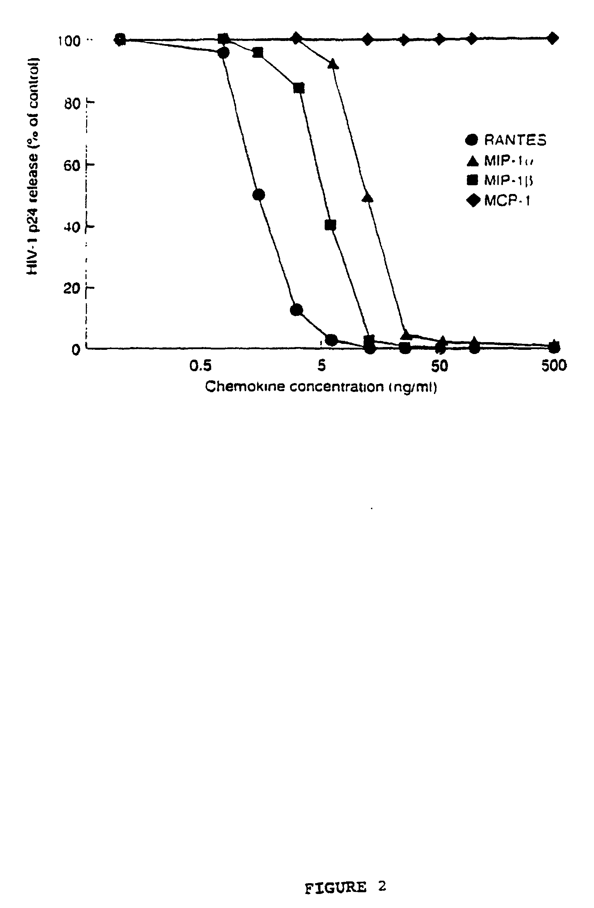

Figure 2 shows dose-dependent inhibition of HIV-1_{BaL} infection in PM1 cells by recombinant human C-C chemokines. The cells were infected with HIV-1. After 3 days, 250 µl of fresh culture medium, containing the appropriate amount of the respective chemokine, were added to each culture. The level of HIV-1 replication was tested at days 5-7 post-infection by p24 antigen capture on cell-free culture supernatant.

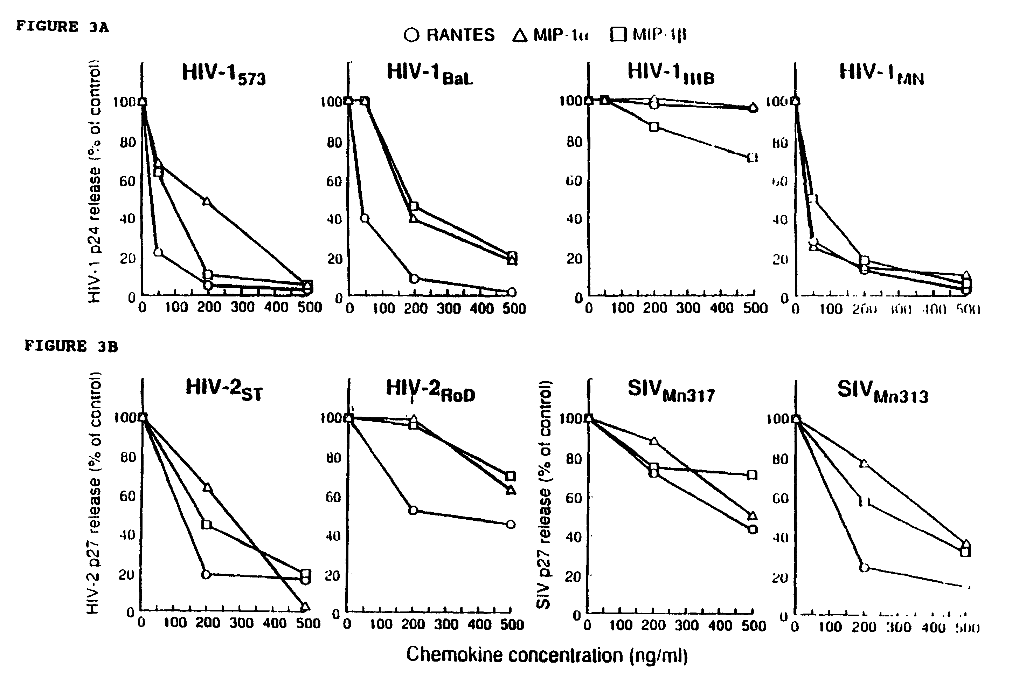

Figures 3A and 3B show the effect of rhRANTES, rhMIP-1α and rhMIP-1β on infection of activated PBMC by different HIV-1 isolates (**A**) or HIV-2 and SIV isolates (**B**). (The "rh" prefix denotes recombinant human.) HIV-1₅₇₃ is a primary isolate grown exclusively in primary PBMC (for less than 5 *in vitro* passages); HIV-1_{BaL} was passaged several times exclusively in primary adherent macrophage cultures derived from adult peripheral blood; HIV-1_{MN} and HIV-1_{mB} are long-term laboratory-passaged isolates, grown in H9 cells. HIV-2_{ROD} and HIV-2_{ST} were grown in a continuous CD4 positive T-cell line, SupT1. The two SIV isolates (SIV_{Mn313} and SIV_{Mn317}) were directly obtained from *in vitro*-activated monkey PBMC cocultivated with human PBMC. The HIV-1 and SIV viral stocks were previously tested for their content of retroviral core antigen and used at 0.25-1 ng of p24 (for HIV-1) or p27 (for SIV) per 10⁶ cells. The HIV-2 viral stocks were tested for reverse transcriptase (RT) activity and used at 4, 000 RT counts per 10⁶ cells, Viral replication was tested by antigen capture ELISA on cell-free culture supernatant collected at days 4-9 post-infection.

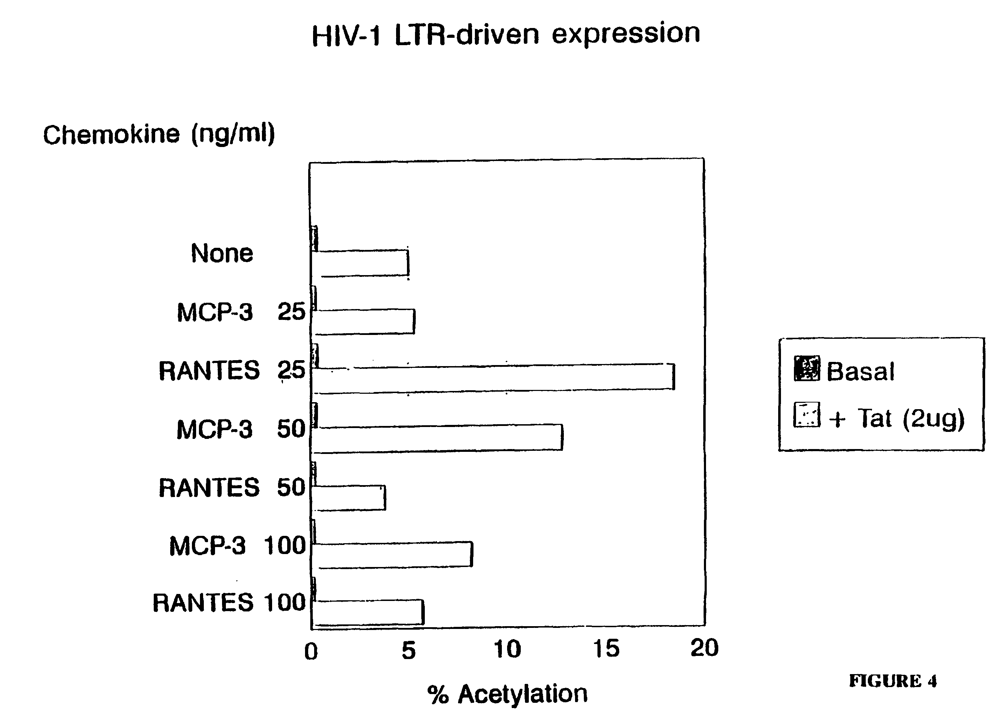

Figure 4 shows the effect of RANTES, and, as a control, MCP-3, on expression of CAT in SupT1 cells. The cells were transfected with 10 µg of a plasmid construct containing CAT functionally linked to the HIV-1 promoter, LTR. In parallel experiments, some cell aliquots were cotransfected with another construct which expresses tat, a transactivator of HIV-1 LTR. Transfection was performed by electroporation of an aliquot of cells (10⁷) in 0.5 ml of culture media, at 260 V, 960 µF. After three days, the CAT activity was measured spectrophotometrically in cytoplasmic extracts.

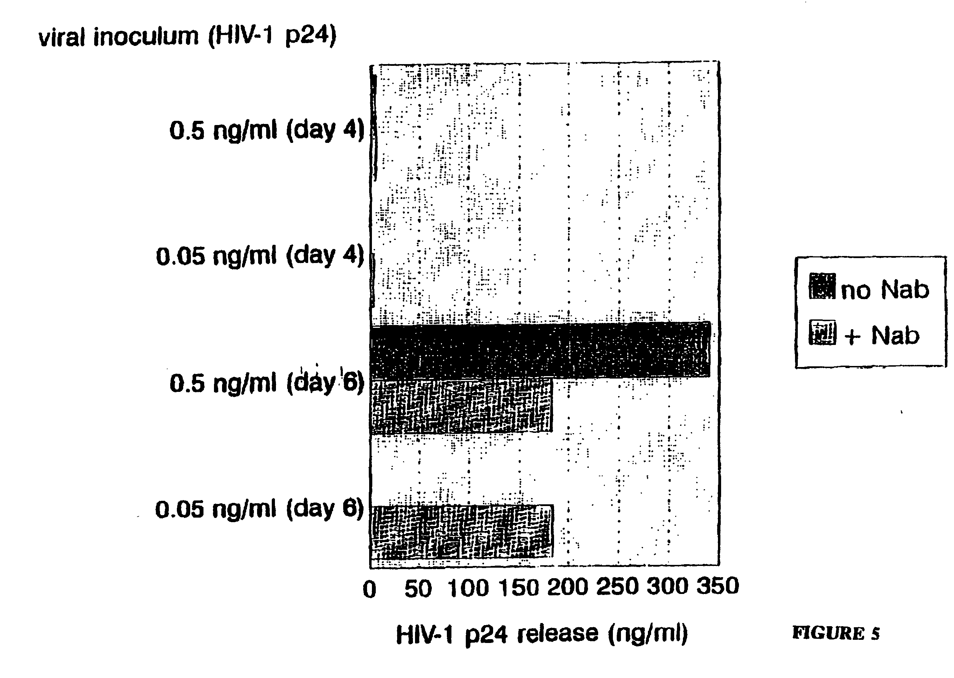

Figure 5 shows the effect of Nabs on transmission of HIV-1 to activated PBMC. Two concentrations of virus were used, 0.5 ng/ml and 0.05 ng/ml. The effect of Nabs (a mixture against RANTES, MIP-1α and MIP-1β) is best seen at condition where the viral load is limited, both after 4 days and after 6 days of infection. Viral transmission was measured as p24 release.

### DETAILED DESCRIPTION OF THE PREFERRED EMBODIMENTS

The present inventors have discovered that RANTES, MIP-1α and MIP-1β, three members of the C-C chemokine subfamily, are HIV-SFs produced by human cells. Instrumental to this observation was the designing of a sensitive and reproducible test system for HIV-SF.

An *in vitro* HIV-SF test is described which is based on an isolated CD4 positive T-cell clone (PM1) which is susceptible to both primary and macrophage-tropic HIV-1 isolates. See Lusso *et al., J. Virol.* **69:** 3712 (1995). In this test system, PM1 cells are infected with HIV-1_{BaL}, the first described macrophage-tropic HIV-1 isolate. See Gartner *et al.*, *Science* **233:** 215 (1986). The level of virus replication in this test is assessed by measuring the release of extracellular p24 core antigen at different days post-infection. Because this test system relies on *de novo* infection with HIV, it is referred to in the present description as "the acute test system." The PM1/HIV-1_{BaL} acute test system is reproducible and highly sensitive to HIV-SF. This allows quantitation of SF in the supernatant of producing cells.

A second development critical for HIV-SF identification was the generation of an efficient producer cell system. Any one CD8 positive cell line that is a high SF producer can lead to a pure cell clone producing high levels of SF. Cloning is effected by a limiting dilution technique, at 0.5 cells/well. The clones are grown to confluence and their cell-free supernatant is tested for HIV-SF production, and a CD8 positive clone producing high levels is isolated. See Example 1 for details on isolation of one high SF producing clone. The above protocol led to identification of a human T-cell leukemia/lymphotropic virus type I (HTLV-I)-immortalized CD8 positive T-cell clone (FC36.22) producing high levels of extracellular HIV-SF.

Any of a number of purification protocols, known to one skilled in the art, can be employed to isolate the factor. The acute infection test system described above serves as an assay for the purification steps. Thus, the development of a sensitive and reliable assay, as well as the isolation by the methods described above of a cell clone (FC36.22) that produces high level of SF are instrumental to, and enable, the purification of SF. To produce material for further analysis, the purification protocol must yield sufficient quantities of homogeneous material. For a review of protein purification techniques, see Scopes, PROTEIN PURIFICATION: PRINCIPLES AND PROCEDURE, 2d ed. (Springer-Verlag, 1987), and Parvez *et al.,* 1 PROGRESS IN HPLC (Science Press, 1985).

Three homogeneous HPLC protein peaks with potent HIV-inhibitory activity were observed from supernatant of FC36.22. To identify the SF contained in those peaks, the proteins were subjected to partial proteolytic degradation and analyzed by partial amino acid sequences. See Lane *et al.*, *J. Protein Chem.* **10**: 151 (1991), and Hunt *et al.*, *Science* **255**: 1261 (1992). Sequence analysis identified them as portions of human RANTES, MIP-1α, and MIP-1β, respectively. By enzyme immunoassay, both clone FC36.22 and *in vitro*-activated CD8 positive T cells from asymptomatic HIV-infected patients were found to produce high levels of extracellular RANTES, MIP-1α, and MIP-1β.

RANTES, MIP-1α, and MIP-1β produced by clone FC36.22 or by patient derived CD8 positive T cells can be tested in the acute test system by addition of excess amounts of specific neutralizing antibodies (Nab), to determine if these chemokines are indeed responsible for the anti-HIV activity. Antibodies to each of those cytokines are available commercially from a number of sources, such as Sigma Immunochemicals (St. Louis, MO), R&D Systems (Minneapolis, MN), and PEPROTECH (Rocky Hill, NJ). The HIV-inhibitory activity produced by either clone FC36.22 or by patient-derived CD8 positive T cells is blocked completely by a combination of specific Nab to RANTES, MIP-1**α** and MIP-1β. But individual Nabs to RANTES or MIP-1α or MIP-1 β have little or no effect on neutralizing SF activity (see Figure 1A and 1B). These data demonstrate that the HIV-SF activity produced by FC36.22 and by in vitro-activated CD8 positive T cells of HIV-infected patients is primarily, if not exclusively, due to the combined effects of RANTES, MIP-1α and MIP-1β. Recombinant RANTES, MIP-1α and MIP-1β are widely available commercially, for example, from Sigma Immunochemicals, R&D Systems, and PEPRO. These chemokines, but not the related chemokine MCP-1, induce a dose-dependent inhibition of infection by HIV-1 virus in PM1 cells (Figure 2).

This result and the other *in vitro* data detailed above indicate that all three chemokines contribute to the physiological anti-HIV effect. At concentrations as shown in Figure 2, however, the factors are effective individually. The effective concentrations shown in Figure 2 are much higher than the concentration for MIP-1α previously reported as effective. See Canque *et al.*, *supra* (1995).

While the mechanism of anti-HIV action for any of the subject chemokines is unclear, it has been shown that they may bind the same cellular receptors, albeit with different affinities. See Schall (1991), *supra.* Thus, effective treatment may be achieved by a combination of the three chemokines. According to one embodiment of the present invention, treatment with any combination of one or more of those chemokines is feasible.

It is expected that RANTES, MIP-1α and MIP-1β can act as antiviral agents with respect to other HIV-1 strains and other retroviruses. Susceptibility has been tested for different HIV-1 strains, including two primary isolates (HIV-1₅₇₃ and HIV-1_{BaL}), never previously passaged in continuous cell lines, and two laboratory isolates (HIV-1_{mB} and HIV-1_{MN}) grown in the CD4 positive T-cell line H9. The tests were performed in primary peripheral blood mononuclear cells (PBMC) activated in vitro with phytohemagglutinin (PHA), which contain both T cells and mononuclear phagocytes; that is, the physiological target cells for HIV-1 infection *in vivo.* Treatment with rhRANTES, rhMIP-1α or rhMIP-1β induced a dose-dependent inhibition of infection by HIV-1₅₇₃, HIV-1_{BaL} and HIV-1_{MN}.

Differences were observed in the relative activity of the chemokines on each viral isolate. In all cases, RANTES was more effective than MIP-1α and MIP-1β. In contrast, HIV-1_{mB}, a long-term laboratory-passaged isolate, was virtually insensitive to the inhibitory effect of the chemokines in PBMC, at the doses tested. See Figure 3A. Other retroviruses were also tested. As shown in Figure 3B, rhRANTES, rhMIP-1α and rhMIP-1β inhibited, in a dose-dependent fashion, infection of primary human PBMC by two HIV-2 and two SIV isolates. Similar results were obtained with the FC36.22 culture supernatant.

Also investigated was the effect of the three recombinant chemokines and the FC36.22 culture supernatant on other, unrelated viruses, including two DNA human herpesviruses (HHV-6 and HHV-7) and an oncoretrovirus (HTLV-I). See Salahuddin *et al.*, *Science* **234**: 596 (1986), Lopez *et al., J. Infect. Dis.* **157**: 1271 (1988), and Lusso *et al., J. Exp. Med.* **166**: 1659 (1988). Neither the chemokines nor the FC36.22 supernatant were able to inhibit infection by either HHV-6 subgroup A (strain GS), HHV-6 subgroup B (strain Z29) or HHV-7 (strain AL) in human cord blood mononuclear cells (CBMC). Similarly, the level of replication of HTLV-I, as assayed by MT-2 cocultivation assay was not reduced by treatment with either rhRANTES or FC36.22 culture supernatant. The lack of susceptibility of HTLV-I to the C-C chemokines also was suggested by the fact that the HTLV-I-immortalized CD4 positive and CD8 positive T-cell lines tested were found to release high levels of extracellular HTLV-I p24 antigen.

Thus, RANTES, MIP-1α and MIP-1β have an inhibitory effect on growth of primary HIV-1 isolates as well as on HIV-2 and SIV. Furthermore, it is demonstrated that RANTES, MIP-1α and MIP-1β were ineffective against some unrelated viruses, such as HTLV-I (an oncoretrovirusy and HHV-6 (A and B) and HHV-7 (HHV-6 and HHV-7 DNA T-lymphotropic human herpesviruses). These specific viral strains may be insensitive to suppression by chemokines, so that testing of a wider panel of viruses and viral isolates will reveal a broader spectrum of viruses which are sensitive to RANTES, MIP-1α and MIP-1β. The effectiveness of RANTES, MIP-1α and MIP-1β treatments, in the dosage ranges described herein above have now been confirmed by others. See Schmidtmayerova *et al., Nature* **382**: 767 (1996), and Vicenzi *et al.* (abstract), 3rd CONF. RETRO AND OPPORT. INFECT., page 163 (1996).

As noted above, CAF reportedly blocks viral transcription. The effect of RANTES, MIP-1α and MIP-1β on viral transcription was tested. One way that this can be accomplished is to employ a viral promoter/reporter gene system which is transduced into human cell lines. Thus, a reporter system is linked to the HIV-1 promoter, *i.e.*, a portion of LTR which normally controls expression of viral RNA. The reporter used can be a chloramphenicol acetyltransferase (CAT) gene, for example. The activity of CAT, thus under the control of HIV-1 LTR, can be monitored in cell extracts via standard protocols, whereby acetylation of chloramphenicol is followed spectrophotometrically or on chromatographic systems. See Maniatis, MOLECULAR CLONING: A LABORATORY MANUAL, (Cold Spring Harbor Laboratory, 1989).

Pursuant to this approach, both crude supernatant from FC36.22 cells and recombinant C-C chemokines were tested. No inhibition of the reporter gene was observed. These results, together with experiments which showed by Northern blot analysis the absence of viral RNA in chemokine-treated cells, clearly indicate that the effect of the HIV-suppressive chemokines occurs at a pre-transcriptional level. See Example 5 below for further experimental details.

By contrast, several groups have showed that the C-C chemokine receptor CKR5, which serves as the principal cellular receptor for RANTES, MIP1α, and MIP1β, is an efficient co-receptor for macrophage-tropic isolates of HIV-1. See Combadiere *et al*., *J*. *Leukocyte Biology* **60**: 147 (1996) ; Samson, *et al.,* , *Biochemistry* **35** : 3362 (1996); Dragic *et al*., *Nature* **381**: 667 (1996); Alkhatib *et al*., *Science* **272:** 1955 (1996); Choe *et al., Cell* **85:** 1135 (1996); Doranz *et al., Cell* **85:** 1149 (1996); Deng, et *al., Nature* **381**: 661 (1996); Murphy, *Ann. Rev. Immunol*. **12**: 593 (1994). The closely related CKR2-B and CKR3 molecules are believed to also be receptors for RANTES, MIP-1α and MIP-1β and can also act as co-receptors for some rare HIV-1 strains. See Doranz *et al.* (1996) and Deng *et al*. (1996), *supra*.

The optimum effective dose of those chemokines when administered individually or in combinations to a patient, can only be estimated at this time. The ED₉₅ in the experiments performed to date in various cell systems and against different viral isolates, suggest a large difference in dosage requirement. A range of 1-1000 ng/ml in platelet free plasma should suffice for any strain of HIV, in different tissues. It is estimated that, in order to achieve those concentrations, delivery of between 1-1000 µg/kg of body weight is required. This should allow the achievement of active concentrations of these chemokines in blood and tissues. However, it must be emphasized that chemokines tend to aggregate in large polymers; thus, the doses reguired could be substantially reduced if a monomeric form of each molecule can be administered. Furthermore, it is predicted that the combined use of two or three chemokines would allow a decrease of the concentrations required for each chemokine.

Purified RANTES, MIP-1α and MIP-1β from any source can be employed for therapeutic purposes. They can be purified from CD8 positive culture supernatant as disclosed above. But a preferred embodiment would take advantage of the genes being known, cloned and expressed in heterologous systems. See schall (1991), *supra,* for a review.

Thus, it may be advantageous to express and purify those chemokines from any expression systems, such as another mammalian cell, a yeast cell, a bacterium, and an insect or insect cell infected or transformed via a baculovirus vector, or plants. Expression in some systems over others may be desirable, for example, because of concerns about proper disulfide bridge formation. With regard to expression and subsequent purification from other expression systems, certain modifications of the gene sequence would be desirable. Those changes may differ for each expression system. Examples of such useful changes are promoters and ribosomal binding sites compatible with the expression system to be used, introduction of appropriate intron sequences, codon usage as preferred in that expression system, replacing the secretory signal sequence to one used by that expression system, or removing such secretory sequences. Addition of short amino acid (aa) "tags" or other protein fusion to allow for more convenient protein purification is an example of yet another useful modification of the gene.

Except for the addition of an aa tag, the foregoing, illustrative changes are at the gene level and would result in a mature gene product (that is, after protein processing by the host cell) that is substantially the same as the mammalian mature RANTES, or MIP-1α, or MIP-1β. The molecular biology techniques to be employed are well known to one skilled in the art. They would consist in large part of subclonings, site-directed mutagenesis, PCR, addition of nucleic acids tails by polymerases or deletions by exonucleases, and sequencing. These techniques are described, for example, by Ausubel *et al*., CURRENT PROTOCOLS IN MOLECULAR BIOLOGY, Vols. 1 and 2 (1987), with supplements, and Maniatis *et al., supra.* Yet other technical references are known and easily accessible to one skilled in the art; see also the description below for site-directed mutagenesis procedures.

Another type of proposed changes to the mammalian RANTES, MIP-1α, or MIP-1β genes actually results in changes to the aa sequence of the mature protein. As described above, those chemokines induce multiple cellular responses, including an inflammatory response related to lymphocyte trafficking regulation, and the desired SF response. In the context of treatment with purified factor, the inflammatory response could amount to a undesirable side-effect.

By routine experimentation, however, one can identify the domains of the mature protein that are responsible for the inflammatory response and the desired SF response, and alter the gene sequence such that the induction of the inflammatory response is reduced, while not reducing the desired SF response. Such altered forms of a protein are generally referred to as "analogs." An analog within the present invention thus is a protein in which at least 70% of the amino acid sequence, preferably 80%, and more preferably 85%, matches the amino acid sequence of the chemokine. The analog of the present invention preferably does not induce the organism's inflammatory response. Guidance as to regions where alteration of the sequence is most likely to provide a useful analog can be obtained by comparing chemokine sequences and identifying conserved and variable regions. See, for example, Baggiolini *et al., Adv. in Immunol.* **55**: 97 (1994).

The experimentation associated with preparing an analog of the present invention could well involve, in addition to the methodologies already described, the technique of site-directed mutagenesis, followed by phenotypic testing of the altered gene product. Some of the more commonly employed site-directed mutagenesis protocols take advantage of vectors that can provide single stranded as well as double stranded DNA, as needed.

Generally, a suitable mutagenesis protocol with such vectors entails the synthesis of a mutagenic primer, i.e., a primer that is complementary to the sequence to be changed but that includes one or a small number of altered, added, or deleted bases. The primer is extended *in vitro* by a DNA polymerase and, after some additional manipulations, the now double-stranded DNA is transfected into bacterial cells. By a variety of methods, the desired mutated DNA that can be identified, and the desired protein can be purified from clones containing the mutated sequence. For longer sequences, additional cloning steps are often required because long inserts (longer than 2kb) are unstable in those vectors. Protocols are known to one skilled in the art and kits for site-directed mutagenesis are widely available from biotechnology supply companies, for example from Amersham Life Science, Inc. (Arlington Heights, IL) and Stratagene Cloning Systems (La Jolla, CA).

RANTES, MIP-1α, and MIP-1β are particularly suitable candidates for such alteration. Namely, they are encoded by small genes, which somewhat limits the number of mutations that have to be tested. Furthermore, the short gene sequence is well suited for the mutagenesis protocols described above. The acute test system described above is also crucial, as it allows testing of the recombinant modified protein for maintenance of its anti-HIV activity. There are various possible approaches to assess the pro-inflammatory activity of modified chemokines. For example, they can be tested for the ability to induce chemotactic response of primary T cells or monocytes *in vitro* in Boyden chamber experiments. See Di Sabato, *Methods in Enzymology* **162**: 717 (1988); Schall et al., *Nature* **347**: 669 (1990). Another method that can be used is the evaluation of the foot-pad inflammatory response in rodents *in vivo.* Modified chemokines can be studied in parallel to the native forms and differences in their inflammatory potency can be documented. See Wolpe *et al*., *J. Exp. Med.* **167**: 570 (1988).

A RANTES analog with the features described above has been produced. The analog is a deletion mutant, missing eight N-terminal amino acids which are present in RANTES itself. The RANTES analog (a) retains high affinity for chemokine receptor, (b) continues to block HIV replication in lymphocytes, and (c) lacks the chemotactic and leukocyte-activating properties of the original RANTES, thus eliminating what could potentially be an Undesirable side-effect of treatment with RANTES. See Arenzana-Seisdedos *et al*., *Nature* **383**: 400 (1996).

According to one embodiment of the present invention, viruses of the retrovirus family are inhibited by gene therapy which involves the introduction of copies of the gene into the patient. Depending in part on the method by which gene(s) for those chemokines are to be introduced in the patient, substantial modification of the gene(s) is contemplated. Thus, tissue-specific promoters or inducible promoters, including the HIV LTR promoter itself, may be introduced. Furthermore, the coding sequence may be altered to reduce the inflammation inducing activity of the gene(s) , as discussed above. Alternatively, especially if the genes are to be used as a prophylactic approach to infection, the gene(s) can be engineered for Weak, constitutive expression.

The gene would be introduced into the organism by any of a few well known approaches. For example, stem, T-lymphocytes, or macrophage cells could be transformed *ex-vivo* with a genetic construct encoding the chemokines or chemokine analogs of the invention. Transformants containing the gene are selected, typically, by cotransfection of a drug resistance marker and selection of transformed cells on a proper growth media. Selected cells are reintroduced into the patient. According to a preferred embodiment, those cells would be derived from the patient itself, if virus-free cells are available. For prophylactic usage of the gene, viral-free cells would be very easy to obtain. Alternatively, the gene could be introduced *in vivo*, part of a viral or plasmid vector. Such vectors are well-known in the art. Some of the viral vectors are engineered to be replication-defective, whereby only a limited number of cells would end up incorporating the viral genome, and thus the gene of interest, into the cellular genome.

According to one embodiment of the present invention, treatment of retroviral disease is prophylactic, in the sense that purified RANTES, MIP-1α, or MIP-1β, or the respective gene as described above, is introduced into a subject before infection. But such administration can follow infection and precede disease development. In particular, it is envisioned that administration could follow shortly after an individual engages in behavior that may expose such individual to the viral agent.

The introduction of the drug into a patient can be via any pharmaceutically acceptable vector. The pharmaceutical vehicles include aerosols, ointments (vaginal, oral, rectal), injections and liposomes. With regard to delivery of the gene for expression in humans, either therapeutically or prophylactically, the pharmaceutically acceptable vehicle could include, for example, a liposome which may be tagged by a tissue-specific ligand, an adenovirus or other attenuated viral vector, a plasmid clone, a ligand-DNA conjugate, or *ex vivo*-transduced human cells, such as stem cells, which are (re) introduced into the patient. Alternatively, delivery of cells engineered with genes encoding RANTES, MIP-1α, or MIP-1β can be accomplished via implantation of a hollow fiber device comprised of "immunoexclusion membranes." See Monaco, *Transplant Proc.* **25**: 2234-36 (1993), and Culver in GENE THERAPY (Mary Ann Liebert Publ. Co., 1994).

Neutralizing antibodies (Nab) for RANTES, MIP-1α, and MIP-1β can be used to improve the isolation of viruses sensitive to those chemokines from patient samples. The isolation of certain viruses, including retroviruses, typically involves the cocultivation of *in vitro* activated lymphocytes obtained from infected individuals with similarly activated lymphocytes from healthy subjects. The activated lymphocytes, however, secrete chemokines that would interfere with transmission of the virus. Accordingly, addition of Nab to those chemokines will improve viral rescue, providing a valuable tool for isolating virus.

To demonstrate the feasibility of this approach, PBMC cells were infected with HIV-1_{BaL}. The viral load factor was 0.5 ng/ml. Cell aliquots were treated, or left untreated, with a mixture of Nabs against RANTES, MIP-1α, and MIP-1β. Viral transmission was measured by p24 antigen release after four or six days of infection. Figure 5 shows the results. As Figure 5 below shows, use of Nab allows observation of viral expression, especially in a situation when the viral load is low.

Monitoring the presence of RANTES, MIP-1α, or MIP-1β either by use of a variation on the acute test system described hereinbefore, or by use of antibodies specific for any one of the chemokines, may allow gathering of prognostic, vaccine efficacy, or diagnostic information. It can be established experimentally that the lever of production for any one of those chemokines changes with disease progression, and that the concentrations present at various times of disease correlate with prognosis.

The present invention is further described by reference to the following examples, which are illustrative only.

### Example 1. Test and producer systems for HIV-SF

The HIV-SF test system was standardized in 48-well microliter plates using PM1 cells acutely infected with HIV-1_{BaL}, the first described macrophage-tropic HIV-1 isolate which displays biological properties resembling those of non-syncytia-inducing primary isolates. See Lusso *et al.* (1995), *supra*, and Gartner *et al.* (1986), *supra.* PM1 cells (2X10⁵/test) were. infected with HIV-1_{BaL} (4 ng of p24 antigen/10⁶ cells) for 2 hr at 37°C, then washed three times with pre-warmed phosphate buffered saline (PBS) and resuspended in complete culture medium (250 µl) containing different dilutions of cell-free culture supernatant from the test cell lines. At least four untreated controls, resuspended in complete medium, with or without exogenous IL-2, were always handled in parallel to treated cultures. At day 3 post-infection, 250 µl of fresh culture medium containing the same original concentration of the respective test supernatant were added to each culture. Five to nine days post-infection, the cultures were harvested, centrifuged to remove the cells and tested for HIV-1 p24 antigen by a commercial ELISA test (Coulter, Hialeah, FL). The culture supernatant tested for the presence of HIV-SF were collected from the cell lines grown at full confluence (2.0.-2.5×10⁶ cells/ml) by centrifugation and filtration through a 0.2-µ membrane. The supernatants were often stored frozen at -70°C before use and discarded after one freeze/thaw cycle. The level of virus replication was assessed by measuring the release of extracellular p24 core antigen at different days post-infection. The PMI/HIV-1_{BaL} test system is highly sensitive to the effect of the HIV-SF contained in the culture supernatant of *in vitro*-activated CD8 positive T cells derived from asymptomatic HIV-infected patients (see below).

Four CD8 positive T-cell lines (see Table 1), three of which were immortalized with human T-cell leukemia/lymphotropic virus type I (HTLV-I), see Gallo, *Nature Med.* **1**: 753 (1995), were screened for their ability to produce extracellular HIV-SF. Two CD4 positive T-cell lines, one infected with HTLV-I (MT-2) and the other with HTLV-II (Vev-II), were also tested as controls. The HTLV-I-negative, CD8-positive T-cell line (PF382) previously was shown to exert a suppressive effect on the growth and differentiation of bone-marrow precursor cells. Pegoraro *et al., J. Natl. Cancer Inst.* **75**: 285 (1985); Bellone *et al., Leukemia* **1**: 603 (1987). PF382 had no detectable effect on HIV replication (see Table 1), while all three HTLV-I-immortalized, CD8-positive T-cell lines were found to produce extracellular HIV-SF, albeit at different levels.

The best producer was 67-I, the culture supernatant of which suppressed HIV-1 infection by more than 95% (95% effective dose, ED₉₅) at 5% (vol/vol). Surprisingly, low levels of HIV-SF activity (ED₉₅ = 50% vol/vol) were also detected in the culture supernatant of MT-2 (CD4 positive/CD8 negative), whereas Vev-II had no significant effect. The culture supernatant from 67-I was tested by specific enzyme immunoassay (EIA) for the presence of several cytokines which are known to exert a suppressive effect on HIV, namely, interferon (IFN)-α, IFN-γ and interleukin (IL)-10. See Mackewicz & Levy (1992), *supra.* None of these cytokines was present in significant amounts.

To obtain a high HIV-SF-producer cell clone, two HTLV-I-immortalized, CD8-positive T-cell lines, CD8-UI and 67-I, were cloned by the limiting dilution technique, at 0.5 cells/well, in the presence of exogenous IL-2. The clones were grown to confluence and their cell-free supernatant was tested for HIV-SF production. A remarkable functional heterogeneity was observed among different clones. The results obtained with representative clones from each cell line are presented in Table 1. One of the two clones with the highest HIV-SF activity, designated FC36.22 (ED₉₅ = 0, 2% vol/vol) , was selected for subsequent studies.

**Table 1.**

| Production of HIV-SF by human T cell lines and clones, as assessed by the PM1/HIV-1_{BaL} test | | |
|---|---|---|
| Cell Type | Membrane Phenotype | HIV-SF **(**ED₉₅^{*}) |
| PF-382 | CD8⁺ | |
| CD8-PI | CD8⁺ | + (20%) |
| CD8-UI | CD8⁺ | + (10%) |
| 67-I | CD8⁺ | + (5%) |
| MT-2 | CD4⁺ | + (50%) |
| Vev-II | CD4⁺ | - |
| | | |

| CD8-UI clones: | | |
|---|---|---|
| B4 | CD8⁺ | + (20%) |
| B7 | CD8⁺ | + (5%) |
| D2 | CD8⁺ | + (5%) |
| D7 | CD8⁺ | + (10%) |
| E2 | CD8⁺ | + (1%) |
| | | |

| 67-I clones: | | |
|---|---|---|
| FC36.4 | CD8⁺ | + (5%) |
| FC36.5 | CD8⁺ | + (1%) |
| FC36.7 | CD8⁺ | + (5%) |
| FC36.12 | CD8⁺ | + (0.2%) |
| FC36.22 | CD8⁺ | + (0.2%) |

| | | |
|---|---|---|
| *ED₉₅ = 95% effective dose (percent of culture supernatant inducing suppression of HIV-1 p24 antigen release equal to or greater than 95%, compared to untreated controls) | | |

### Example 2. Purification and identification of HIV-SF

Clone FC36.22 was grown to full confluence in a serum-free medium, HB-101, (Irvin Scientific, Santa Ana, CA) which was supplemented with rhIL-2 (10 ng/ml). Four liters of cell-free culture supernatant were collected. The supernatant volume was concentrated to 250 ml by means of a Millipore prep/scale tangential flow filtration cartridge (0.09 M²) having a 30 Kd nominal molecular weight exclusion limit. The concentrate then was applied to Centriprep-50 centrifugal concentrators with a 50 Kd nominal molecular weight exclusion limit. The concentrate (30 ml) was diluted twofold with PBS and reapplied to the Centriprep-50 concentrator. The final Centriprep-50 concentrate was then applied to a concentrator having 100 Kd nominal molecular weight exclusion limit. The concentrate obtained was diluted twofold with PBS and reapplied to the Centriprep-100 concentrator to yield a final concentrate of 15 ml.

Aliquots of all the concentrates and the filtrates were tested, at various dilutions, for HIV-SF activity. Significant activity was detected in the final 15 ml concentrate obtained from Centriprep-100. The concentrate was then clarified by centrifugation at 100, 000xg for 60 min at 4°C, diluted threefold in 10 mM Tris-HCl pH 8.0 and applied to a 250x10 mm ID, weak-anion exchange HPLC column, marketed under the trade name "Synchropak" by Phenomenex Inc., TORRANCE, CA., which had been equilibrated in 10 mM Tris-HCl, pH 8.0. Proteins bound to the column were eluted with a linear gradient of 0→1 M NaCl in 10 mM Tris-HCl, pH 8.0, at a flow rate of 1 ml/min.

The fractions thus obtained, as well as the original material loaded onto the column and the flow-through, were tested at various dilutions for HIV-SF activity. The bulk of the activity was recovered between 0.3 and 0.4 M NaCl. The fractions displaying inhibitory activity were pooled and brought to pH 2.0 by addition of trifluoroacetic acid (TFA) and subjected to reversed-phase HPLC. The acidified pool was applied to a 3.9 x 300 mm analytical µBondapak C-18 column (Waters Instruments), equilibrated in H₂O containing 0.1% TFA. Proteins bound to the column were eluted with a 5-min linear gradient of aqueous acetonitrile (0→25%) containing 0.1% TFA. After 10 min at 25% acetonitrile/TFA, the column was further developed with a 60 min linear gradient of 25→65% aqueous acetonitrile/TFA. The flow rate was maintained at 1 ml/min.

The fractions collected were tested at various dilutions for HIV-SF activity. Three major peaks of activity were recovered. Fractions corresponding to each of the peaks were pooled, diluted twofold in H₂O with 0.1% TFA and reapplied to the analytical C-18 column. The column was developed with a 30 min linear aqueous acetonitrile gradient (0→65%) containing 0.1% TFA at a flow rate of 1 ml/min. The HIV-inhibitory activity coincided in each cases with a single peak eluted from the column.

The proteins corresponding to each of the three peaks were subjected to proteolytic digestion. See Lane *et al.*, *J. Protein Chem.* **10**: 151 (1991); Hunt *et al.*, *Science* **255**: 1261 (1992). Three distinct peptide fragments from the first protein, two from the second, and two from the third were sequenced. All the sequences obtained were considered of high-confidence quality. The three amino-acid sequences obtained from protein 1 matched those of three different portions of human RANTES, an 8 kilodalton (Kd) peptide belonging to the C-C or β-chemokine subfamily. See Schall, *Cytokine* **3**: 165 (1991). The two sequences from the second protein matched those of two different portions of human MIP-1α, another 8 Kd C-C chemokine, highly related to RANTES. *Id*. The two sequences from the third protein matched those of two portions of human MIP-1β. *Id*.

### Example 3. Effect of recombinant RANTES, MIP-1α and MIP-1β on cellular proliferation

To rule out that the antiviral activity of RANTES, MIP-1α and MIP-1β could be due to a negative effect on cellular viability or proliferation, we tested the effect of these chemokines on the proliferative response of primary human PBMC to stimulation with either PHA or monoclonal antibody OKT3. Only MIP-1α exerted a dose-dependent inhibitory effect, albeit very limited, on [³H]-Thymidine incorporation by OKT3-stimulated cells (24.4%, 14.1% and 2.0% inhibition at 500, 100 and 20 ng/ml, respectively) (Table 2). However, it had no significant effect on PHA-stimulated cells. The other chemokine-treated cultures displayed values of [³H]-Thymidine incorporation between 87% and 105% of the control. None of the chemokines had any stimulatory effect on resting, non-mitogen-treated cells (Table 3).

Similar data were obtained with supernatant from the HTLV-I-immortalized CD8 positive T-cell lines (data not shown). These results indicate that the antiviral activity of RANTES, MIP-1α and MIP-1β is not due to suppression of cellular proliferation. This notion is also supported by the observation of higher levels of cellular viability in infected PM1 cultures treated with HIV-inhibitory doses of chemokines, compared to untreated controls.

**Table 2.**

| Incorporation of [³H]-Thymidine by human PBMC stimulated for 72 hr with OKT3 or PHA, in the presence or absence of C-C chemokines | | | |
|---|---|---|---|
| Treatment | Unstimulated | OKT3 | PHA |
| None | 405^{*} | 46515 | 52472 |
| | | | |

| RANTES | | | |
|---|---|---|---|
| 500 ng/ml | 308(76.0%) | 40865 (87.8%) | 47268 (90.1%) |
| 100 ng/ml | 299 (73.8%) | 45572 (98.0%) | 49032 (93.4%) |
| 20 ng/ml | 368 (90.8%) | 47715 (104.8%) | 47360 (90.3%) |
| | | | |

| MIP-1α | | | |
|---|---|---|---|
| 500 ng/ml | 261 (64.4%) | 35182 (75.6%) | 48878 (93.1%) |
| 100 ng/ml | 438 (108.1%) | 39969 (85.9%) | 45460 (86.6%) |
| 20 ng/ml | 389 (96.0%) | 45614 (98.0%) | 46052 (87.8%) |
| | | | |

| MIP-1β | | | |
|---|---|---|---|
| 500 ng/ml | 378 (93.3%) | 42344 (91.0%) | 46014 (87.7%) |
| 100 ng/ml | 389 (96.0%) | 43087 (92.6%) | 47987 (91.4%) |
| 20 ng/ml | 278 (68.6%) | 42567 (91.5%) | 45756 (87.2%) |

| | | | |
|---|---|---|---|
| *PBMC were separated by Ficoll gradient centrifugation and placed in round-bottom 96-well plates (10⁵ cells/well). Monoclonal antibody OKT3 (ascites) was used at 1:500; PHA at 1 µg/ml. The values in the Table denote average corrected counts per minute from triplicate cultures; the numbers in parentheses denote the percent radionucleide incorporation, compared to the respective control not treated with chemokines. | | | |

### Example 4. Inhibition of immunodeficiency retroviruses by RANTES, MIP-1α and MIP-1β

To elucidate whether the inhibitory effect of the C-C chemokines was restricted to-HIV-1, the Susceptibility of two related primate retroviruses, HIV-2 and SIV, was studied. As shown in Figure 3B, rhRANTES, rhMIP-1α and rhMIP-1β inhibited, in a dose-dependent fashion, infection of primary human PBMC by two HIV-2 and two SIV isolates.

All three chemokines were remarkably more effective on HIV-2_{ST} (characterized by slow growth rate and low cytopathic effect) than on HIV-2_{ROD} (characterized by rapid growth rate and high cytopathic effect). They also were more effective on SIV_{Mn317} than on SIV_{Mn313}, although these two isolates do not differ significantly in their growth rate and cytopathic potential. Similar results were obtained with the FC36.22 culture supernatant.

Also investigated was the effect of the three recombinant chemokines and the FC36.22 culture supernatant on other, unrelated viruses, including two human herpesviruses (HHV-6 and HHV-7) and an oncoretrovirus (HTLV-I). See Salahuddin *et al.* (1986), *supra;* Lopez *et al.* (1988), *supra;* Lusso *et al.* (1994), *supra.* The level of HHV-6 and HHV-7 infection was assessed by indirect immunofluorescence analysis. See Lusso *et al*. (1988), *supra.* To evaluate the effect of the chemokines on HTLV-I infection, the MT-2 cocultivation test was used. Human CBMC were activated *in vitro* with PHA and then cocultured at 10:1 ratio with MT-2 cells irradiated with 10000 rad, in the presence of rhIL-2 (10 ng/ml). With respect to cell-free supernatant collected at days 2, 4, 6, 8 and 10 after starting the culture, HTLV-I replication was tested for p24 antigen via an ELISA marketed by ABI Advanced Biotechnology Inc. (Columbia, MD). Neither the chemokines nor the FC36.22 supernatant inhibited infection by HHV-6 subgroup A (strain GS), HHV-6 subgroup B (strain Z29), or HHV-7 (strain AL) in human cord blood mononuclear cells (CBMC) (data not shown). Similarly, the level of replication of HTLV-I, as assayed by the MT-2 cocultivation assay described above, was not reduced by treatment with either rhRANTES or the FC36.22 culture supernatant.

### Example 5. The mode of action of RANTES, MIP-1α and MIP-1β

A reporter gene was linked to the HIV-1 promoter, namely, the reporter was linked to a part of the viral LTR which normally controls expression of viral RNA. The reporter used was a CAT gene. The CAT activity was monitored in cell extracts by determining the extent of chloramphenicol acetylation. See Maniatis, supra. Both crude supernatant from FC36.22 cells and recombinant C-C chemokines were tested. Ten micrograms of the HIV-LTR/CAT plasmid DNA were transfected into SupT1 cells and, in some experiments, co-transfected with an expression vector for the Tat protein of HIV-1, which mediates transactivation of the expression of HIV. The transfected cells were cultured, alternatively, in the presence or absence of HIV-SF.

Three days after transfection, cytoplasmic extracts were made and tested for CAT enzymatic activity, that is measured as percent acetylation of the enzyme substrate, chloramphenicol, added to the reaction. A summary of experiments performed with rhRANTES and a related, but not HIV-suppressive chemokine, rhMCP-3, are presented in Figure 4. No inhibition or enhancement of either basal (Tat-independent) and Tat-induced expression of the reporter gene was obtained with other rh chemokines or with the crude FC36.22 supernatant. These results, together with the documented absence of viral RNA in chemokine-treated cells (Northern blot data not shown), indicate that the effect of the HIV-suppressive chemokines occurs at a pre-transcriptional level. This in turn suggests that the C-C chemokines do not act at the same level as the unidentified CAF activity, which reportedly blocks viral replication by inhibiting viral RNA transcription.

## Claims

1. Use of at least one chemokine selected from the group consisting of RANTES, MIP-1α, MIP-1β, and an analog thereof for the preparation of a pharmaceutical composition for the treatment of a retrovirus infection.

2. The use according to claim 1, wherein the patient is a human.

3. The use according to claim 1 or 2, wherein said retrovirus is HIV.

4. The use according to any one of claims 1 to 3, wherein said pharmaceutical composition is administered by injection.

5. The use according to any one of claims 1 to 3, wherein said pharmaceutical composition is administered by liposome delivery.

6. Use of at least one polynucleotide encoding a chemokine selected from the group consisting of RANTES, MIP-1α, MIP-1β, and an analog thereof for the preparation of a pharmaceutical composition for the treatment of a retrovirus infection.

7. The use according to claim 6, wherein said polynucleotide comprises a tissue-specific or an inducible promoter that is linked functionally to a structural sequence coding for said chemokine.

8. The use of any one of claims 1 to 7, wherein said treatment is a prophylactic treatment.

9. An in vitro method of isolating retrovirus obtained from an infected subject, comprising the steps of (A) administering to a sample from said infected subject an antibody that binds RANTES, MIP-1α or MIP-1β and then (B) cocultivating the sample with uninfected lymphocytes.

## Patentansprüche

1. Verwendung von mindestens einem Chemokin, das ausgewählt ist aus der Gruppe bestehend aus RANTES, MIP-1α, MIP-1β und einem Analogon davon, für die Herstellung eines Arzneimittels zur Behandlung einer Retrovirusinfektion.

2. Verwendung nach Anspruch 1, wobei der Patient ein Mensch ist.

3. Verwendung nach Anspruch 1 oder 2, wobei das Retrovirus HIV ist.

4. Verwendung nach einem der Ansprüche 1 bis 3, wobei das Arzneimittel durch Injektion verabreicht wird.

5. Verwendung nach einem der Ansprüche 1 bis 3, wobei das Arzneimittel durch Liposomenzufuhr verabreicht wird.

6. Verwendung mindestens eines Polynucleotids, das ein Chemokin codiert, das ausgewählt ist aus der Gruppe bestehend aus RANTES, MIP-1α MIP-1β und einem Analogon davon, für die Herstellung eines Arzneimittels zur Behandlung einer Retrovirusinfektion.

7. Verwendung nach Anspruch 6, wobei das Polynucleotid einen gewebespezifischen oder induzierbaren Promotor umfasst, der funktionell mit einer Struktursequenz verknüpft ist, die das Chemokin codiert.

8. Verwendung nach einem der Ansprüche 1 bis 7, wobei die Behandlung eine prophylaktische Behandlung ist.

9. In-vitro-Verfahren zur Isolierung eines Retrovirus, der von einer infizierten Person erhalten wurde, wobei das Verfahren die folgenden Schritte umfasst:
(A) Verabreichen eines Antikörpers, der RANTES, MIP-1α oder MIP-1β bindet, einer Probe der infizierten Person und (B) Ko-Züchten der Probe mit nicht-infizierten Lymphocyten.

## Revendications

1. Utilisation d'au moins une chimiokine sélectionnée dans le groupe constitué de RANTES, MIP-1α, MIP-1β, et un analogue de ceux-ci pour la préparation d'une composition pharmaceutique destinée au traitement d'une infection par un rétrovirus.

2. Utilisation selon la revendication 1, dans laquelle le patient est un humain.

3. Utilisation selon la revendication 1 ou 2, dans laquelle ledit rétrovirus est le VIH.

4. Utilisation selon l'une quelconque des revendications 1 à 3, dans laquelle ladite composition pharmaceutique est administrée par injection.

5. Utilisation selon l'une quelconque des revendications 1 à 3, dans laquelle ladite composition pharmaceutique est administrée au moyen de liposomes.

6. Utilisation d'au moins un polynucléotide codant pour une chimiokine sélectionnée dans le groupe constitué de RANTES, MIP-1α, MIP-1β, et un analogue de ceux-ci pour la préparation d'une composition pharmaceutique destinée au traitement d'une infection par un rétrovirus.

7. Utilisation selon la revendication 6, dans laquelle ledit polynucléotide comprend un promoteur spécifique de tissu ou inductible qui est lié de manière fonctionnelle à une séquence structurale codant pour ladite chimiokine.

8. Utilisation selon l'une quelconque des revendications 1 à 7, dans laquelle ledit traitement est un traitement prophylactique.

9. Méthode in vitro pour isoler un rétrovirus obtenu d'un sujet infecté, la méthode comprenant les étapes de (A) administrer à un échantillon dudit sujet infecté un anticorps qui lie RANTES, MIP-1α, ou MIP-1β et ensuite (B) co-cultiver l'échantillon avec des lymphocytes non-infectés.
